# EUROPEAN PATENT APPLICATION

(11) **EP 4 375 667 A1**
(43) Date of publication of application: **29.05.2024**
(21) Application number: 22845777.6
(22) Date of filing: 04.07.2022
(51) Int. Cl.: G01N 33/543

(54) **TEST CARTRIDGE AND METHOD FOR MANUFACTURING TEST STRIP**

(30) Priority: 21.07.2021 JP 2021120871
(71) Applicant: FUJIFILM Corporation, Tokyo 106-8620 (JP)
(72) Inventor: ISHII, Hiroyasu, Ashigarakami-gun, Kanagawa 258-8538 (JP); SATO, Keiichiro, Ashigarakami-gun, Kanagawa 258-8538 (JP)
(74) Representative: Hughes, Andrea Michelle
(86) International application number: PCT/JP2022/026645
(87) International publication number: WO 2023/002843

(57) **Abstract**

An assay cartridge including the assay strip that has, as an assay region for determining whether a sample is positive or negative, a plurality of kinds of assay regions each on which a plurality of kinds of binding substances that respectively specifically bind to a plurality of different kinds of test substances is immobilized, and that includes a strip-shaped carrier capable of performing an assay of a plurality of kinds of assay items depending on the number of different kinds of the assay regions, in which in a case where a direction orthogonal to a longitudinal direction of the strip-shaped carrier is defined as a width direction, the plurality of kinds of the assay regions have a length in the width direction of the strip-shaped carrier shorter than an entire width of the strip-shaped carrier, and the plurality of kinds of the assay regions are arranged to be positionally deviated in the width direction, and a manufacturing method of the assay strip.

## Description

### BACKGROUND OF THE INVENTION

### 1. Field of the Invention

The present disclosure relates to an assay cartridge and a manufacturing method of an assay strip.

### 2. Description of the Related Art

JP2010-261961A and JP2009-058507A describes an immunochromatographic kit for performing an assay of whether a sample is positive or negative, that is, whether or not the sample contains a test substance, using an immunochromatographic method. This immunochromatographic kit is called an assay cartridge or the like. The assay cartridge includes an assay strip including a strip-shaped carrier having an assay region on which a binding substance that specifically binds to a test substance is immobilized. The color development state of the assay region changes, for example, in a case where the sample developed in the assay region includes a test substance.

The assay cartridge includes an assay cartridge having a plurality of kinds of assay regions corresponding to a plurality of kinds of assay items such as influenza A and influenza B on a strip-shaped carrier of one assay strip. In a case where there are two kinds of assay items, the strip-shaped carrier is provided with a first assay region and a second assay region, each on which different test substances in accordance with the assay items are immobilized. According to such an assay cartridge, since a plurality of kinds of assay regions are provided in the strip-shaped carrier of one assay strip, it is possible to perform a plurality of kinds of assays having different assay items at once in one sample.

The arrangement pattern of the plurality of kinds of assay regions described in JP2010-261961A and JP2009-058507A is as follows. First, in a case where a direction orthogonal to the longitudinal direction of the strip-shaped carrier is defined as a width direction, each of the plurality of kinds of assay regions has a line shape extending along the width direction of the strip-shaped carrier. The length of the plurality of kinds of line-shaped assay regions is formed over the entire region in the width direction of the strip-shaped carrier, that is, the length of each assay region is the length corresponding to the entire width of the strip-shaped carrier. In this way, the plurality of kinds of assay regions having the same length as the width of the strip-shaped carrier are arranged to be positionally deviated in the longitudinal direction of the strip-shaped carrier.

### SUMMARY OF THE INVENTION

There is the following improvement demand with respect to an assay cartridge in the related art having an arrangement pattern of a plurality of kinds of assay regions, as described in JP2010-261961A and JP2009-058507A. First, the binding substance that is immobilized to the assay regions is relatively expensive, and as the number of assay regions provided in the strip-shaped carrier increases, the incremental cost also increases. Therefore, even in a case where the assay items are increased, there is a demand to reduce an increase in cost due to the increase in the assay items by suppressing the increase in the amount of the binding substance. In addition, there is also a demand to ensure the identifiability of each assay region even in a case where the area of the assay region is reduced than that of the related art in order to suppress an increase in cost.

An object of the present disclosure is to provide an assay cartridge and a manufacturing method of an assay strip in which an increase in cost can be suppressed as compared with the prior art while ensuring the identifiability of a plurality of kinds of assay regions even in a case where a plurality of kinds of assay regions are provided in a strip-shaped carrier of the assay strip.

The assay cartridge of the present disclosure is an assay cartridge that is used for an immunochromatographic assay, the assay cartridge comprising
an assay strip that has, as an assay region for determining whether a sample is positive or negative, a plurality of kinds of assay regions each on which a plurality of kinds of binding substances that respectively specifically bind to a plurality of different kinds of test substances is immobilized, and that includes at least a strip-shaped carrier capable of performing an assay of a plurality of kinds of assay items depending on the number of different kinds of the assay regions,
in which in a case where a direction orthogonal to a longitudinal direction of the strip-shaped carrier is defined as a width direction,
the plurality of kinds of the assay regions have a length in the width direction of the strip-shaped carrier shorter than an entire width of the strip-shaped carrier, and
the plurality of kinds of the assay regions are arranged to be positionally deviated in the width direction.

In the assay cartridge of the present disclosure, the plurality of kinds of the assay regions are preferably arranged side by side in the width direction of the strip-shaped carrier.

In the plurality of kinds of the assay regions of the assay cartridge of the present disclosure, center positions in the longitudinal direction of the strip-shaped carrier may coincide with each other, and widths are the same.

In the assay cartridge of the present disclosure, different kinds of the assay regions adjacent to each other in the width direction of the strip-shaped carrier may be arranged to be partially deviated in the longitudinal direction of the strip-shaped carrier.

In the assay cartridge of the present disclosure, the plurality of kinds of the assay regions may be arranged in an aspect without overlapping each other in the longitudinal direction of the strip-shaped carrier.

In the assay cartridge of the present disclosure, in the longitudinal direction of the strip-shaped carrier, an interval between end parts of the plurality of kinds of assay regions is preferably less than 2 mm.

The manufacturing method of the assay strip included in the assay cartridge of the present disclosure, the manufacturing method comprising a coating step of applying a plurality of kinds of coating liquids each containing the plurality of kinds of the binding substances to form the plurality of kinds of the assay regions on a sheet-shaped carrier having an area including a plurality of the strip-shaped carriers, in which in the sheet-shaped carrier, the plurality of kinds of the coating liquids are applied in an aspect in which an arrangement pattern of the plurality of kinds of the assay regions in the one strip-shaped carrier is periodically repeated, and
a cutting step of cutting the sheet-shaped carrier along a direction orthogonal to a repetition direction of the arrangement pattern.

In the manufacturing method of the assay strip of the present disclosure, in the coating step, the plurality of kinds of the coating liquids are preferably applied in an aspect in which a plurality of the arrangement patterns is linearly arranged along the repetition direction.

In the manufacturing method of the assay strip of the present disclosure, in the coating step, in the repetition direction, a gap region to which none of the plurality of kinds of the coating liquids is applied may be provided between adjacent arrangement patterns in the repetition direction, and in the cutting step, the sheet-shaped carrier may be cut in the gap region.

In the manufacturing method of the assay strip of the present disclosure, in the coating step, the plurality of kinds of the coating liquids may be applied in an aspect in which adjacent assay regions between adjacent arrangement patterns in the repetition direction are positionally shifted in the direction orthogonal to the repetition direction.

In the manufacturing method of the assay strip of the present disclosure, a masking member including a plurality of opening portions linearly arranged along the repetition direction in a period in which a width of one strip-shaped carrier is defined as one period, in which a length of each of the opening portions in the repetition direction is the length of the assay region, may be used, and
the coating step may include a first coating step of placing the masking member at a first position on the sheet-shaped carrier and applying a first coating liquid that is one of the plurality of kinds of coating liquids on a first part that is exposed from the opening portion, and includes a second coating step of moving the masking member from the first position to a second position by shifting in the repetition direction within the one period and applying a second coating liquid that is another one of the plurality of kinds of coating liquids on a second part that is exposed from the opening portion at the second position and to which the first coating liquid is not applied.

In the manufacturing method of the assay strip of the present disclosure, the plurality of kinds of the coating liquids may be applied by an inkjet method.

The manufacturing method of the assay strip of the present disclosure, including a marking step of providing, on the sheet-shaped carrier, a mark indicating a position to be cut in the cutting step, in which in the cutting step, the sheet-shaped carrier is cut based on the mark.

According to the assay cartridge and the manufacturing method of the assay strip of the present disclosure, an increase in cost can be suppressed as compared with the prior art while ensuring the identifiability of a plurality of kinds of assay regions even in a case where a plurality of kinds of assay regions are provided in a strip-shaped carrier of the assay strip.

### BRIEF DESCRIPTION OF THE DRAWINGS

Fig. 1 is a perspective view of an assay cartridge according to the present disclosure.
Fig. 2 is an exploded perspective view of the assay cartridge according to the present disclosure.
Fig. 3 is a plan view (a) and a side view (b) showing a configuration of an assay strip.
Fig. 4 is an explanatory diagram of an immunochromatographic method.
Fig. 5 is a diagram for describing modification examples of arrangement of assay regions.
Fig. 6 is an explanatory diagram 1 of a manufacturing method of an assay strip.
Fig. 7 is an explanatory diagram 2 of the manufacturing method of an assay strip.
Fig. 8 is an explanatory diagram 3 of the manufacturing method of an assay strip.
Fig. 9 is an explanatory diagram of a case where a cutting position is shifted in the manufacturing method of an assay strip.
Fig. 10 is an explanatory diagram of a modification example of the manufacturing method of an assay strip.
Fig. 11 is an explanatory diagram of a modification example of the manufacturing method of an assay strip.
Fig. 12 is an explanatory diagram of a case where a cutting position is shifted in a case where the manufacturing method of Fig. 11 is used.
Fig. 13 is an explanatory diagram of a modification example of the manufacturing method of an assay strip.
Fig. 14 is an explanatory diagram of a case where a cutting position is shifted in a case where the manufacturing method of Fig. 13 is used.
Fig. 15 is an explanatory diagram of a manufacturing method of an assay strip using an inkjet method.

### DESCRIPTION OF THE PREFERRED EMBODIMENTS

Hereinafter, an assay cartridge of the present disclosure will be described with reference to the drawings. The constituent elements indicated by the same reference numerals in the respective drawings mean the same constituent elements. However, unless otherwise specified in the specification, each constituent element is not limited to one, and a plurality of each constituent element may be present.

In addition, description of overlapping configurations and reference numerals in the respective drawings may be omitted. In addition, the present disclosure is not limited to the embodiments below, and can be implemented with appropriate modifications, such as omitting a configuration or replacing a configuration with a different configuration within the scope of the object of the present disclosure.

The directions indicated by the arrows X and Y, which are appropriately shown in the respective figures, are directions along the horizontal plane and are orthogonal to each other. In addition, the direction indicated by the arrow Z is a direction along the perpendicular direction (vertical direction). The directions indicated by the arrows X, Y, and Z in respective figures coincide with each other.

### <Overview of assay cartridge>

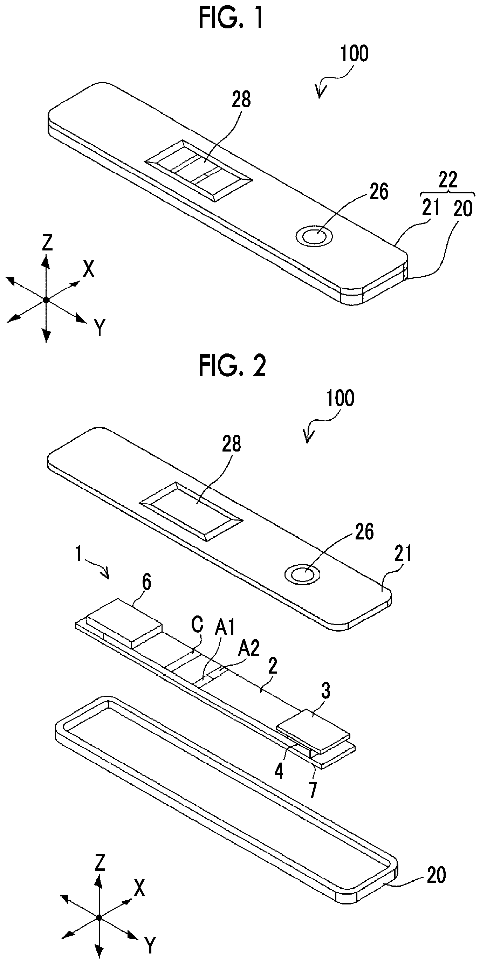

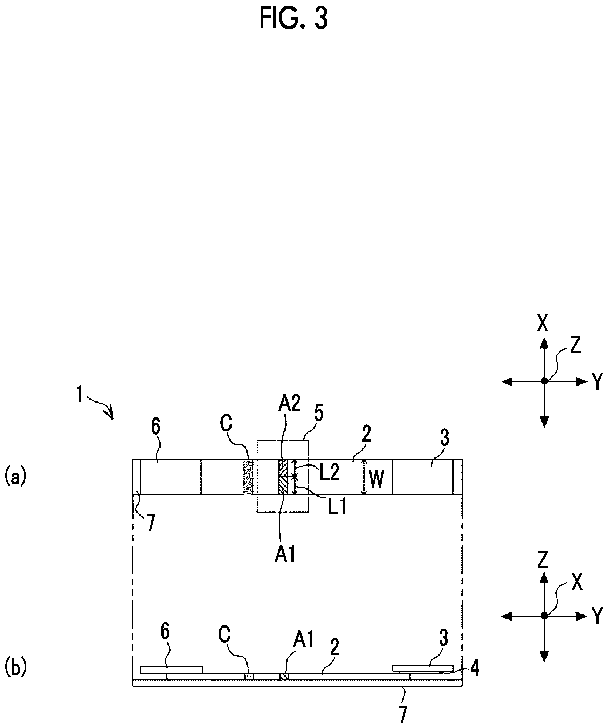

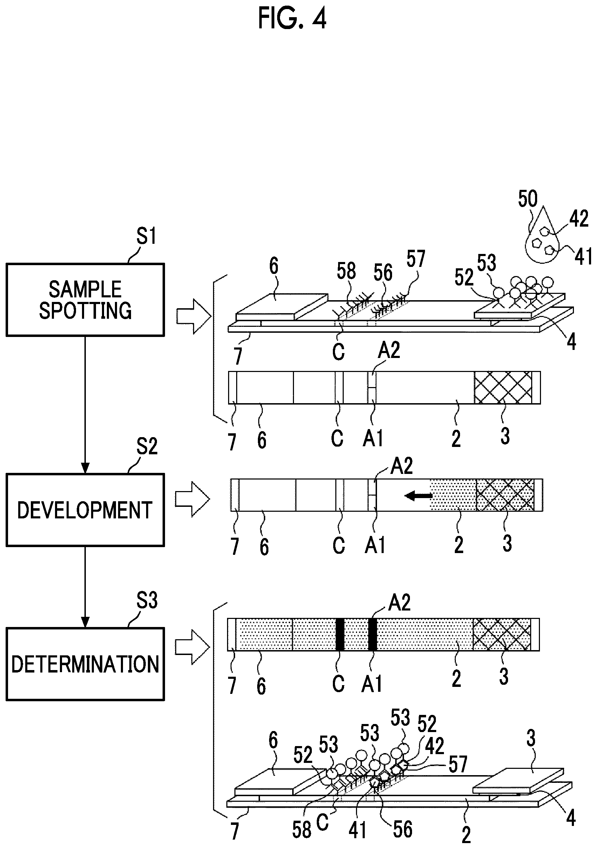

Fig. 1 is an external view of an assay cartridge 100 (Hereinafter, referred to as a cartridge 100) according to an embodiment, and Fig. 2 is an exploded perspective view of the cartridge 100. Fig. 3 is a plan view (a) and a side view (b) of the assay strip included in the cartridge 100. Fig. 4 is an explanatory diagram of an immunochromatographic method.

The cartridge 100 is a single-use type that is used one by one in each sample of assay target. As shown in Fig. 2, an assay strip 1 including a strip-shaped immunochromatographic carrier 2 (hereinafter, referred to as a strip-shaped carrier 2) is included in the cartridge 100. A first assay region A1 and a second assay region A2 is provided in the strip-shaped carrier 2. The first assay region A1 and the second assay region A2 are an example of a plurality of kinds of assay regions each on which a plurality of kinds of binding substances that respectively specifically bind to a plurality of different kinds of test substances are immobilized. The strip-shaped carrier 2 has the first assay region A1 and the second assay region A2, which are different kinds of assay regions, and a plurality of kinds of assay items can be assayed depending on the number (two in the present example) of two kinds of assay regions of the first assay region A1 and the second assay region A2.

For example, the color development state of the first assay region A1 changes in a case where the sample 50 (see Fig. 4) includes the first test substance 41 (see Fig. 4), that is, in a case where the sample 50 is positive with respect to the first test substance 41. Similarly, the color development state of the second assay region A2 changes in a case where the sample 50 includes the second test substance 42 (see Fig. 4), that is, in a case where the sample 50 is positive with respect to the second test substance 42. The first test substance 41 and the second test substance 42 are different kinds of test substances for each other, and the present cartridge 100 is a cartridge that simultaneously determines whether each the sample 50 is positive or negative with respect to each of two different test substances of the first test substance 41 and the second test substance 42. For example, in a case where the first test substance 41 is an antigen of an influenza A and the second test substance 42 is an antigen of an influenza B, the presence or absence of illness in regard to the influenza A and the influenza B can be simultaneously assayed.

The "change in color development state" includes any of an aspect in which a first color different from the color of the strip-shaped carrier 2 changes to another second color (that is, a color change), an aspect in which the color of the strip-shaped carrier 2 changes to another color by developing a color different from that of the strip-shaped carrier 2 (that is, color development), and an aspect in which the density of the color changes (that is, a density change).

The sample 50 is simply required to be a specimen that may contain a test substance, and the sample 50 is not particularly limited. The sample 50 is, for example, a biological specimen, particularly body fluid or excrement of an animal (particularly, a human) such as blood, serum, blood plasma, spinal fluid, tear fluid, sweat, urine, pus, nasal mucus, nasal swab, throat swab, nasal aspirate, or sputum, an organ, a tissue, a mucous membrane and skin, or swabs containing them, a liquid specimen containing animals and plants themselves or a dried body thereof. Examples of the test substance include an antigen, an antibody, a protein, and a low-molecular-weight compound.

The cartridge 100 has a configuration that allows a user to visually confirm whether the sample 50 is positive or negative. Such a cartridge 100 is also referred to as an immunochromatographic assay tool, an immunochromatographic assay kit, or the like.

As shown in Fig. 1 and Fig. 2, as an example, the cartridge 100 includes a case 22 constituted of a case main body 20 and a cover member 21. The case 22 is formed of, for example, a resin material. An opening is formed in an upper part of the case main body 20, and the assay strip 1 is accommodated therein. The cover member 21 covers the opening of the case main body 20 by being attached to the opening part of the case main body 20. The case 22 has an elongated shape as a whole in accordance with the elongated shape of the assay strip 1.

In the present example, a dropping port 26 and an observation window 28 are provided on an upper part of the case 22 constituted of the cover member 21. Each of these parts is integrally molded with the cover member 21 as an example. The dropping port 26 is an opening for adding dropwise a sample 50 into the inside of the case 22.

An observation window 28 is an opening portion for observing the first assay region A1 and the second assay region A2 from the outside, in the present example, the size of the observation window 28 is a size such that, in addition to the first assay region A1 and the second assay region A2, the control region C described below, can also be observed. A transparent member is fitted to the observation window 28. The user can observe the color development state of the first assay region A1, the second assay region A2, and the control region C through the observation window 28.

### <Assay strip>

The assay strip 1 includes a strip-shaped carrier 2, a label holding pad 3, and an absorption pad 6. Then, the strip-shaped carrier 2 is fixedly supported on a substrate 7 provided with a pressure-sensitive adhesive sheet on the surface thereof. A label holding pad 3 is arranged at one end of the longitudinal direction of the carrier 2, and an absorption pad 6 is provided at the other end of the longitudinal direction of the strip-shaped carrier 2. The longitudinal direction of the strip-shaped carrier 2 means the longitudinal direction of the assay strip 1 (the Y direction in the figure). A direction (X direction in the figure) perpendicular to the longitudinal direction of the strip-shaped carrier 2 is referred to as a width direction of the strip-shaped carrier 2. The width of the strip-shaped carrier 2 has the same meaning as the width of the assay strip 1.

The strip-shaped carrier 2 is a porous insoluble carrier for developing a sample 50, and includes the first assay region A1, the second assay region A2, and the control region C. The first assay region A1, the second assay region A2, and the control region C are provided on the strip-shaped carrier 2 and arranged between the label holding pad 3 and the absorption pad 6 in the longitudinal direction of the assay strip 1. The control region C is provided on the absorption pad 6 side with respect to the first assay region A1 and the second assay region A2.

As also shown in Fig. 3, the first assay region A1 and the second assay region A2 are arranged to be positionally deviated in the width direction of the strip-shaped carrier 2. As an example, in the present example, the first assay region A1 and the second assay region A2 are arranged side by side in the width direction of the strip-shaped carrier 2. Further, in the first assay region A1 and the second assay region A2 of the present example, the center positions in the longitudinal direction of the strip-shaped carrier 2 coincides with each other, and widths are the same. Here, the term "same" includes a range of ±10% in addition to perfect same.

In other words, the first assay region A1 and the second assay region A2 of the present example are, as a whole, one row of line extending in the width direction (X direction in the figure) of the strip-shaped carrier 2, and the regions obtained by dividing this line into two regions in the width direction are each the first assay region A1 and the second assay region A2.

In addition, both the length L1 of the first assay region A1 and the length L2 of the second assay region A2 in the width direction of the strip-shaped carrier 2 are shorter than the total width W of the strip-shaped carrier 2. In the present example, the length L1 and the length L2 are the same, and are each a length W/2 which is half the total width W of the strip-shaped carrier 2. That is, L1 = L2 = W/2.

The control region C is a line-shaped region extending in the width direction of the strip-shaped carrier 2.

In Fig. 3, a state in which the first assay region A1, the second assay region A2, and the control region C are expressed as visible lines on the strip-shaped carrier 2 is shown, but these are not always expressed. Details is described below, but before developing the sample 50 (see Fig. 4), the colors of the first assay region A1, the second assay region A2, and the control region C are substantially the same as the color of the strip-shaped carrier 2 (for example, white), and thus the first assay region A1, the second assay region A2, and the control region C cannot be clearly visually recognized at this stage. The first assay region A1 is expressed as a line by the increase of the color optical density in a case where the sample 50 is developed and the developed sample 50 is positive with respect to the first test substance 41. Similarly, the second assay region A2 is expressed as a line by the increase of the color optical density in a case where the sample 50 is developed and the developed sample 50 is positive with respect to the second test substance 42.

The control region C is also expressed as a line by the increase of the color optical density in a case where the sample 50 is developed. Accordingly, the control region C becomes visible.

As the strip-shaped carrier 2, for example, a porous material such as a nitrocellulose membrane can be used. In addition, the substrate 7 on which the strip-shaped carrier 2 is fixed has a pressure-sensitive adhesive surface to which the strip-shaped carrier 2 is attached.

As shown in Fig. 4, a labeling substance 53 is immobilized to the label holding pad 3. The labeling substance 53 is modified with the labeling binding substance 52 that specifically binds to the first test substance 41 and the second test substance 42 contained in the sample 50. The label holding pad 3 is fixed on the strip-shaped carrier 2 at a position facing the dropping port 26 (see Fig. 2) of the cover member 21. Therefore, the sample 50 is added dropwise onto the label holding pad 3 from the dropping port 26. Therefore, the label holding pad 3 corresponds to a spotting region on which the sample 50 is spotted.

The label holding pad 3 is fixed at one end of the strip-shaped carrier 2 in the longitudinal direction via a double-sided tape 4. As the labeling substance 53, it is possible to use, for example, a gold colloidal particle having a diameter of 50 nm (EM. GC50, manufactured by BBI Solutions). The labeling substance 53 is not limited to the gold colloid, and a metal sulfide that can be used in a general chromatographic method, a coloring particle that are used in an immunoagglutination reaction, or the like can be used, where a metal colloid is particularly preferable. Examples of the metal colloid include a gold colloid, a silver colloid, a platinum colloid, an iron colloid, an aluminum hydroxide colloid, and a composite colloid thereof. In particular, at an appropriate particle diameter, a gold colloid is preferable since it exhibits a red color, a silver colloid is preferable since it exhibits a yellow color, the gold colloid is most preferable among them.

As shown in Fig. 4, the first assay region A1 includes a first binding substance 56 that specifically binds to the first test substance 41 and captures the first test substance 41. In the first assay region A1, in a case where the first test substance 41 is captured by binding the first binding substance 56 to the first test substance 41, the labeling binding substance 52 bound to the first test substance 41 and the labeling substance 53 are captured. In a case where the first test substance 41 is included in the sample 50, the first test substance 41 and the labeling substance 53 are captured in the first assay region A1, and thus the color optical density in the first assay region A1 is increased to be not less than a preset reference. In this way, the first assay region A1 is a region for confirming the presence or absence of the first test substance 41 by a labeling signal from the labeling substance 53 captured via the first test substance 41. Similarly, the second assay region A2 includes a second binding substance 57 that specifically binds to the second test substance 42 and captures the second test substance 42. In the second assay region A2, in a case where the second test substance 42 is captured by binding the second binding substance 57 to the second test substance 42, the labeling binding substance 52 bound to the second test substance 42 and the labeling substance 53 are captured. In a case where the second test substance 42 is included in the sample 50, the second test substance 42 and the labeling substance 53 are captured in the second assay region A2, and thus the color optical density in the second assay region A2 is increased to be not less than a preset reference. In this way, the second assay region A2 is a region for confirming the presence or absence of the second test substance 42 by a labeling signal from the labeling substance 53 captured via the second test substance 42.

The control region C includes a confirming binding substance 58 that specifically binds to the labeling binding substance 52, and captures the labeling substance 53 via the labeling binding substance 52. In a case where the sample 50 is spotted on the label holding pad 3, the labeling substance 53 that is bound to neither the first test substance 41 nor the second test substance 42 among the labeling substances 53 modified with the labeling binding substance 52 is developed in the strip-shaped carrier 2 together with the sample 50. The labeling substance 53 that is bound to neither the first test substance 41 nor the second test substance 42 is captured in neither the first assay region A1 nor the second assay region A2 and passes through the first assay region A1 and the second assay region A2. The labeling substance 53 that has passed through the first assay region A1 and the second assay region A2 is captured in the control region C via the labeling binding substance 52 by binding the labeling binding substance 52 to the confirming binding substance 58. The labeling substance 53 is captured in the control region C, and thus the color optical density of the control region C is increased to be not less than a preset reference. The control region C is a region for confirming the completion of the development of the sample 50 in the first assay region A1 and the second assay region A2 by the labeling signal from the labeling substance 53 captured via the labeling binding substance 52. Therefore, the control region C may be referred to as a confirmation region.

### (Binding substance)

The labeling binding substance 52 that modifies the labeling substance 53 and specifically binds to the first test substance 41 and the second test substance 42 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like.

The first binding substance 56 that is immobilized in the first assay region A1 and specifically binds to the first test substance 41 is a substance that specifically binds to the test substance, for example, in a case where the first test substance 41 is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like.

The second binding substance 57 that is immobilized in the second assay region A2 and specifically binds to the second test substance 42 is a substance that specifically binds to the test substance, for example, in a case where the second test substance 42 is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like.

The first binding substance 56 specifically binds to the first test substance 41, but does not specifically bind to the second test substance 42. Similarly, the second binding substance 57 specifically binds to the second test substance 42, but does not specifically bind to the first test substance 41. For example, in a case where the first test substance 41 is an influenza A virus and the second test substance 42 is an influenza B virus, the first binding substance 56 is an antibody for an influenza A virus, which does not react with the influenza B virus, the second binding substance 57 is an antibody for an influenza B virus, which does not react with the influenza A virus, or the like.

The confirming binding substance 58 that specifically binds to the labeling binding substance 52 may be the first test substance 41 and the second test substance 42 themselves or may be a compound having a site recognized by the labeling binding substance 52. Examples thereof include a compound obtained by binding a derivative of the first test substance 41 or the second test substance 42 to a protein, and the like.

### (Absorption pad)

The absorption pad 6 absorbs the sample 50 developed on the strip-shaped carrier 2. The label holding pad 3 is provided at one end of the strip-shaped carrier 2, and the absorption pad 6 is provided at the other end of the strip-shaped carrier 2, which is downstream in the development direction of the sample to be added dropwise on the label holding pad 3. The absorption pad 6 absorbs the sample 50 that has passed through the first assay region A1 or the second assay region A2.

The absorption pad 6 is formed of a porous material and absorbs the sample 50 from the strip-shaped carrier 2 by a capillary force. By absorbing the sample 50 at the end part of the strip-shaped carrier 2, the sample 50 is suppressed from staying in the strip-shaped carrier 2, and the sample 50 can be smoothly flowed toward the absorption pad 6.

### <Immunochromatographic method>

An immunochromatographic method is described with reference to Fig. 4. Here, a case where the sample 50 includes the first test substance 41 and the second test substance 42, that is, on the premise that the sample 50 is positive with respect to both the first test substance 41 and the second test substance 42 is described.

First, the sample 50 is spotted on the label holding pad 3 which is the spotting region (Step S 1). The first test substance 41 and the second test substance 42 in the sample 50 spotted on the label holding pad 3, specifically binds to the labeling binding substance 52 that modifies the labeling substance 53 contained in the label holding pad 3. By the capillary action in the strip-shaped carrier 2, in the strip-shaped carrier 2, the sample 50 is developed from the label holding pad 3 toward the assay region A (Step S2).

The first test substance 41 in the sample 50 that has reached the first assay region A1 is captured by the first binding substance 56 in the first assay region A1. That is, the labeling substance 53 bound to the first test substance 41 via the labeling binding substance 52 is captured in the first assay region A1. Similarly, the second test substance 42 in the sample 50 that has reached the second assay region A2 is captured by the second binding substance 57 in the second assay region A2. That is, the labeling substance 53 bound to the second test substance 42 via the labeling binding substance 52 is captured in the second assay region A2. On the other hand, the labeling substance 53 that is not bound to the first test substance 41 and the second test substance 42 passes through the first assay region A1 and the second assay region A2 without being captured and is captured by the confirming binding substance 58 in the control region C. As a result, the densities of the first assay region A1, the second assay region A2, and the control region C are increased, and the lines of the first assay region A1, the second assay region A2, and the control region C are expressed. Therefore, it becomes in a state in which it can be determined (Step S3).

The assay procedure according to the immunochromatographic method using the cartridge 100 of the present example is as described above. According to the cartridge 100, since a plurality of different kinds of assay regions that each capture test substances different from each other, such as the first assay region A1 and the second assay region A2, are provided, the positive-negative determination of the plurality of test substances can be carried out at the same time.

As described above, in of the assay strip 1 provided in the cartridge 100, the length L1 of the first assay region A1 and the length L2 of the second assay region A2 in the width direction of the strip-shaped carrier 2 are shorter than the total width W of the strip-shaped carrier 2. In addition, the first assay region A1 and the second assay region A2 are arranged to be positionally deviated in the width direction. In a case where the length L1 of the first assay region A1 and length L2 of the second assay region A2 in the width direction are made shorter than the total width W of the strip-shaped carrier 2, it is possible to suppress an increase in the amount of respective binding substances for forming the first assay region A1 and the second assay region A2 as compared with the prior art in which the lengths L1 and L2 are the same length as the total width W. Therefore, even in a case where the number of assay items are increased, it is possible to suppress an increase in cost as compared with the prior art.

In addition, in a case where the assay region is formed in a line shape extending in the width direction, examples of the method for suppressing the amount of the binding substance also include a method of narrowing the line width. However, in a case where the line width of the assay region is too narrow, there is a concern that the identifiability may be significantly lowered and the assay accuracy may be lowered. On the other hand, in the cartridge 100 of the present example, it is considered that the decrease in the detection accuracy is small because the length of the assay region in the width direction of the assay strip 1 is shortened without reducing the line width of the assay region.

Furthermore, since the first assay region A1 and the second assay region A2 are arranged to be positionally deviated in the width direction, the identifiability is good as compared with the case where the positions coincide. This is because, for example, in a case where, in the longitudinal direction of the assay strip 1, a plurality of kinds of assay regions A1 and A2 are close to each other or at least a part thereof overlaps each other, it is difficult to identify respective assay regions A1 and A2 in a case of coinciding the positions in the width direction. On the other hand, it is easy to identify it in a case where the positions of the first assay region A1 and the second assay region A2 in the width direction deviate from each other.

In the present example, although the length L1 of the first assay region A1 in the width direction of the assay strip 1 and the length L2 of the second assay region A2 in the width direction of the assay strip 1 are the same, both the lengths may not be the same. However, in a case where one length is shorter than the other length, the visibility of the positive-negative determination for one assay region is reduced, and thus the lengths of the assay strip 1 in the width direction of the plurality of assay regions are preferably about the same.

In addition, the first assay region A1 and the second assay region A2 are arranged side by side in the width direction of the strip-shaped carrier 2. Accordingly, the following effects are achieved. That is, in the assay cartridge in the related art, the plurality of kinds of assay regions having the same length as the width of the strip-shaped carrier are arranged to be positionally deviated in the longitudinal direction of the strip-shaped carrier. In a case where a plurality of kinds of assay regions are arranged to be positionally deviated in the longitudinal direction of the strip-shaped carrier, in order not to cause the confusion for the assay result of the assay item, they are arranged separately at least 2 mm or more in the longitudinal direction of the strip-shaped carrier not to interfere each other. Therefore, in a case where there are a plurality of kinds of assay regions as compared with the case where there is one assay region, there is a problem that the development region of the sample is lengthened, the liquid feeding time is extended accordingly, and thus the assay time is lengthened.

On the other hand, in the assay strip 1 of the present embodiment, the first assay region A1 and the second assay region A2 are arranged side by side in the width direction of the assay strip 1. Therefore, the positions of the first assay region A1 and the second assay region A2 in the longitudinal direction of the assay strip 1 are substantially the same. Since the sample 50 is often developed along the longitudinal direction of the assay strip 1, the development time of the sample 50 in the respective assay regions A1 and A2 is substantially the same. Accordingly, even though there are a plurality of kinds of assay items, the assay can be performed in substantially the same time as in a case where there is one kind of assay item.

In addition, in the first assay region A1 and the second assay region A2 of the assay strip 1 according to the present embodiment, the center positions in the longitudinal direction of the assay strip 1 coincides with each other, and widths are the same. Therefore, since the development time until the sample 50 is developed in each of the first assay region A1 and the second assay region A2 is substantially the same, even in a case of assaying a plurality of kinds of assay items, the assay can be performed in an assay time equivalent to that in a case where there is one kind of the assay item. In addition, since the widths and the positions of the different assay regions are common, it is also easy to perform the manufacture.

### <Modification example of arrangement of assay region>

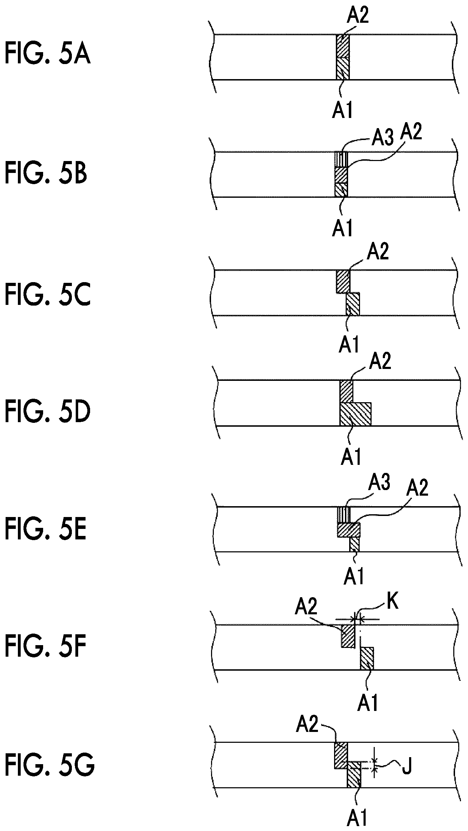

With reference to Fig. 5, a modification example of the arrangement of the assay region is shown. Fig. 5A to Fig. 5G are schematic diagrams showing the arrangement of the assay regions A1 to A3 on the strip-shaped carrier 2 of the assay strip 1. Each figure shown in Fig. 5 is an enlarged view showing a partial region 5 of the strip-shaped carrier 2 surrounded by a two-dot chain line in the plan view of the assay strip 1 of Fig. 3. Fig. 5A shows the arrangement of the assay regions A1 and A2 in the case of the above-described embodiment described with reference to Fig. 2 to Fig. 4, and Fig. 5B to Fig. 5G show modification examples of the arrangement of the assay regions.

In the above-described assay strip 1, as shown in Fig. 5A, the strip-shaped carrier 2 includes two assay regions A1 and A2. However, for example, as shown in Fig. 5B, the assay strip 1 of the present disclosure may include three or more assay regions A1 to A3. The assay strip 1 may further include four or more assay regions.

Also in a case where three or more kinds of assay regions are provided, similarly to the case where there are two assay regions, since an increase in the amount of the binding substance for forming the assay region can be suppressed by making the respective lengths of the plurality of kinds of assay regions shorter than the total width of the assay strip, an increase in cost can be suppressed as compared with the prior art even in a case where the assay items are increased. In addition, since the plurality of kinds of the assay regions are arranged to be positionally deviated in the width direction, the identifiability is good as compared with the case where the positions in the width direction coincide.

In the example shown in Fig. 5A, the first assay region A1 and the second assay region A2 are arranged in a state where the center positions thereof coincide with each other in the longitudinal direction of the strip-shaped carrier 2. In the present disclosure, the "arrangement in the width direction" as shown in Fig. 5C, maintains a state where at least a part of respective assay regions A1 and A2 overlays is maintained and includes a state where the center positions in the longitudinal direction are deviated from each other, in addition to the configuration of Fig. 5A.

In addition, at this time, in the longitudinal direction of the assay strip 1, not only the case where the widths of the respective assay regions A1 and A2 are the same, but also the plurality of assay regions A1 and A2 in Fig. 5D or the plurality of assay regions A1 to A3 in Fig. 5E may have the widths different from each other.

Since the plurality of kinds of assay regions A1 and A2, or A1 to A3 provided on one assay strip 1 are arranged side by side in the width direction of the assay strip 1, the development time of the sample 50 in each of the assay regions A1 and A2, or A1 to A3 is substantially the same in a state where the center positions of the respective assay are deviated from each other, but a part of the respective assay regions overlap. Accordingly, even though there are a plurality of kinds of assay items, the assay can be performed in substantially the same time as in a case where there is one kind of assay item.

In addition, as shown in Fig. 5C, in a case where different kinds of assay regions adjacent to each other in the width direction of the assay strip 1 are arranged to be partially deviated in the longitudinal direction of the assay strip 1, there is also a case where it is good in terms of the identifiability of the adjacent assay regions.

Furthermore, as shown in Fig. 5F and Fig. 5G, the plurality of kinds of assay regions A1 and A2 may be arranged in an aspect without overlapping each other in the longitudinal direction of the assay strip 1. As the positions of the plurality of kinds of assay regions A1 and A2 are separated from each other in the longitudinal direction, the identifiability is improved.

In a case where a plurality of kinds of assay regions A1 and A2 are arranged in an aspect without overlapping in the longitudinal direction of the assay strip 1, the interval K between the end parts of the plurality of kinds of assay regions A1 and A2 is preferably less than 2 mm. In the related art, in a case where a plurality of assay regions is each formed in a line shape in the longitudinal direction of the assay strip 1 over the entire region in the width direction, the assay regions are arranged with an interval of 2 mm or more such that the adjacent assay regions are not interfered with each other. In a case where the interval K between the end parts of the plurality of kinds of assay regions A1 and A2 is less than 2 mm, the assay time can be shortened as compared with the case where the adjacent assay regions in the related art has been arranged at an interval of 2 mm or more.

In Fig. 5A to Fig. 5G, a plurality of assay regions A1 to A3 included in one assay strip 1 are arranged not to overlap in the width direction of the assay strip 1. However, as in Fig. 5G, the plurality of assay regions A1 and A2 may partially overlap in the width direction of the assay strip 1. Also in this case, since the plurality of assay regions A1 and A2 have a part that do not overlap with each other in the width direction, the effect of improving the identifiability can be obtained.

The above-described cartridge 100 has a specification that makes it possible to determine whether the sample 50 is positive or negative by spotting the sample 50. The specification of the cartridge of the present disclosure is not limited thereto, and may be configured such that a developing liquid for promoting the development of the sample 50 is held and the developing liquid can be developed after the sample 50 is spotted. In addition, the cartridge of the present disclosure may be configured that an amplifying liquid for amplifying labeling signals of the assay region and the control region is held and the amplifying liquid can be fed after the spotting of the sample 50.

Although the assay strip 1 includes the strip-shaped carrier 2, the label holding pad 3, and the absorption pad 6, according to the specifications of the cartridge, a liquid feeding pad for feeding a reagent such as a developing liquid, an amplifying liquid, or the like may be further included.

### <Manufacturing method of assay strip>

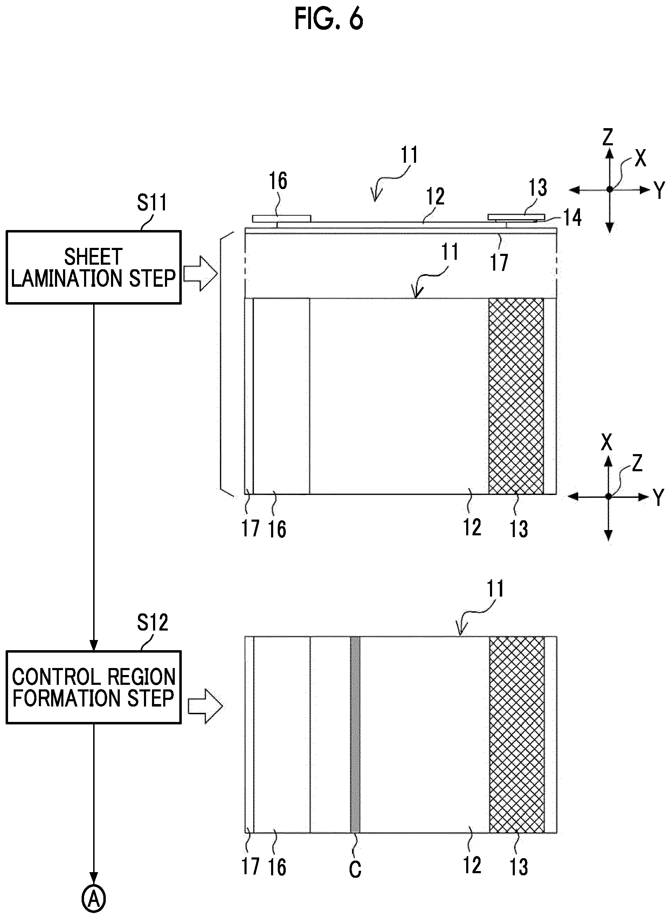

As shown in Fig. 6 to Fig. 8, the manufacturing method of the above-described assay strip 1 includes a coating step for forming a plurality of kinds of assay regions A1 and A2 on a sheet-shaped carrier 12 having an area including a plurality of assay strips 1, and a cutting step for cutting the sheet-shaped carrier 12. The coating step is a step of applying a plurality of kinds of coating liquids each including a plurality of kinds of binding substances to the sheet-shaped carrier 12, and a step of applying the plurality of kinds of coating liquids in an aspect in which the arrangement pattern P of the plurality of kinds of the assay regions in one assay strip 1 is periodically repeated in the sheet-shaped carrier 12. The cutting step is a step of cutting the sheet-shaped carrier 12 along a direction orthogonal to a repetition direction of the arrangement pattern P of the plurality of kinds of the assay regions.

Hereinafter, a specific manufacturing method of the assay strip 1 will be described with reference to Fig. 6 to Fig. 8.

In a case of manufacturing the assay strip 1, first, as shown in Fig. 6, as a sheet lamination step (S11), a sheet-shaped carrier 12 is attached on a sheet-shaped substrate 17 having an area including the plurality of the assay strips 1, and the sheet-shaped label holding pad 13 and the sheet-shaped absorption pad 16 are laminated on the sheet-shaped carrier 12 to prepare a sheet-shaped laminate 11. The sheet-shaped carrier 12 has an area including a plurality of strip-shaped carriers 2. Similarly, the sheet-shaped label holding pad 13 has an area including a plurality of the label holding pads 3. The sheet-shaped label holding pad 13 is attached to the sheet-shaped carrier 12 via a sheet-shaped double-sided tape 14. In the sheet-shaped label holding pad 13, a labeling substance 53 (see Fig. 4) modified with a labeling binding substance 52 is immobilized in advance.

Next, as a control region forming step, a control region C is formed at a predetermined position on the absorption pad 6 side on the surface of the sheet-shaped carrier 12 in the sheet-shaped laminate 11 (Step S12). The control region C is formed by applying a confirming binding substance 58 (see Fig. 4).

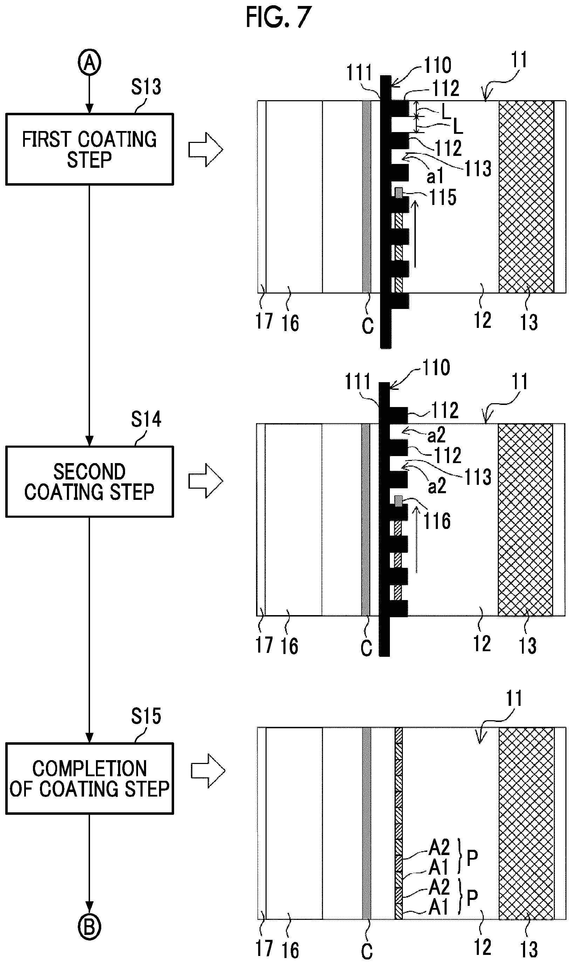

Next, the above-described coating step for forming a plurality of kinds of assay regions (here, the first assay region A1 and the second assay region A2) is carried out. As shown in Fig. 7, in the present example, the coating step includes a first coating step (Step S13) and a second coating step (Step S14). In the coating step, a masking member 110 is used (see Fig. 7A). The masking member 110 includes a linear support member 111 and a plurality of mask portions 112 arranged in a comb-teeth shape with respect to the support member 111. In the mask portion 112, the width in the longitudinal direction of the support member 111 is the length L (= L1 = L2) in the width direction of the strip-shaped carrier 2 (see Fig. 3) of the first assay region A1 and second assay region A2, and a width of the opening portion 113 between the mask portions 112 is also L. That is, the masking member 110 includes a plurality of opening portions 113 that are linearly arranged along the repetition direction in a period having a width W of one strip-shaped carrier 2 as one period. The length of each opening portion 113 in the repetition direction is the length L of the first assay region A1 and the second assay region A2.

As shown in Fig. 7, in the first coating step, at a first position where the mask portion 112 covers a portion where the second assay region A2 is formed and the opening portion 113 is located at a portion where the first assay region A1 is formed, the masking member 110 is placed on the sheet-shaped carrier 12 and a coating head 115 holding the first coating liquid containing the first binding substance 56 (see Fig. 4) is moved in parallel with the longitudinal direction of the support member 111 (Step S13). In this way, by applying the first coating liquid to the first part a1 of the sheet-shaped carrier 12, where the coating head 115 is exposed to the opening portion 113 of the masking member 110, the first assay region A1 is formed.

Next, a second coating step (Step S14) is carried out. As shown in Fig. 7, in the second coating step (Step S14), the masking member 110 is deviated on the sheet-shaped carrier 12 by a length L in the longitudinal direction of the support member 111 and moved from the first position to a second position where the opening portion 113 is located at a position where the second assay region A2 is formed. In this manner, in a state where the masking member 110 is placed on the sheet-shaped carrier 12, the coating head 116 holding the second coating liquid containing the second binding substance 57 (see Fig. 4) is moved in parallel with the longitudinal direction of the support member 111. In this way, by applying the second coating liquid to the second part a2, where the first coating liquid is not applied, of the sheet-shaped carrier 12, where the coating head 116 is exposed to the opening portion 113 of the masking member 110, the second assay region A2 is formed.

As described above, the applying is performed in an aspect in which the plurality of arrangement patterns P is linearly arranged along the repetition direction of the arrangement pattern P of the plurality of kinds of the assay regions (here, the first assay region A1 and the second assay region A2) in one assay strip, and the coating step is completed (Step S15). Accordingly, the sheet-shaped carrier 12 in which the arrangement pattern P of the first assay region A1 and the second assay region A2 in one assay strip 1 shown in Fig. 3 is linearly periodically repeated is obtained (see Fig. 7). In the present example, a repetition direction of the arrangement pattern P coincides with the width direction of the sheet-shaped carrier 12 corresponding to the width direction of the strip-shaped carrier 2.

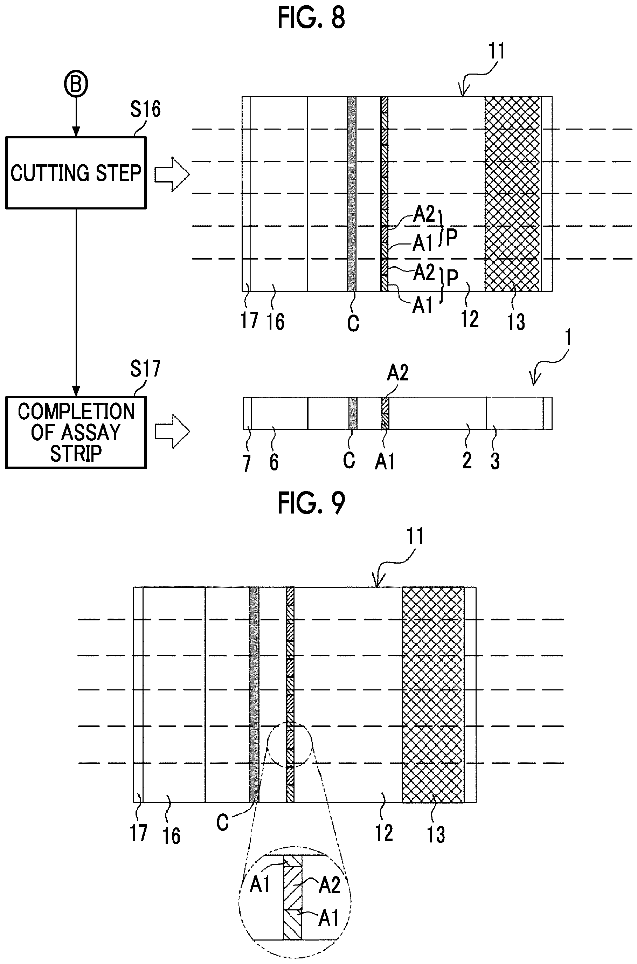

Next, in the cutting step (Step S16) shown in Fig. 8, the sheet-shaped laminate 11 including the sheet-shaped carrier 12 in which the first assay region A1 and the second assay region A2 are formed is cut. In Fig. 8, a portion to be cut (hereinafter, referred to as a cutting position) is indicated by a broken line. The cutting is performed with the width of one assay strip 1 including one arrangement pattern P including one first assay region A1 and one second assay region A2, to obtain a plurality of assay strips 1. The cutting position is a boundary between the arrangement patterns P, and in the present example, is a boundary between the first assay region A1 and the second assay region A2. The sheet-shaped laminate 11 is cut at a cutting position in a period in which a width of one strip-shaped carrier 2 is set as one period. After determining the first one cutting position and cutting, a position where the sheet-shaped carrier 12 is shifted by one period is the next cutting position.

According to the above procedure, the manufacturing step of the assay strip 1 provided in the assay cartridge 100 described with reference to Fig. 1 to Fig. 4 can be terminated to obtain the assay strip 1 (Step S17).

Since each of the assay regions A1 and A2 is applied and formed using the masking member 110, the formation region of each of the assay regions A1 and A2 can be easily defined, and each of the assay regions A1 and A2 can be formed. The lengths in the width direction of the strip-shaped carrier 2 of the respective assay regions A1 and A2 are shorter than the total width of the strip-shaped carrier 2.

In the above description, the case where the two assay regions A1 and A2 are applied and formed by using the single masking member 110 and moving the masking member 110 has been described, but a plurality of masking members 110 may be used to apply and form the respective assay regions. In particular, in a case where the application length or the application width differs between a plurality of kinds of assay regions, application and formation of the plurality of kinds of assay regions can be efficiently realized by using a plurality of masking members 110 having opening portions of sizes corresponding to the respective assay regions.

### <Modification example of manufacturing method>

Modification examples of the above-described manufacturing method are described with reference to Fig. 9 to Fig. 15. In Fig. 9 to Fig. 15, the same constituent elements as those of the constituent elements shown in Fig. 1 to Fig. 8 are denoted by the same reference numerals, and detailed description thereof is omitted.

As shown in Fig. 8, in the assay strip 1 obtained according to the above-described manufacturing method, the first assay region A1 and the second assay region A2 having the same length in the width direction of the strip-shaped carrier 2 are provided linearly in parallel. However, since the regions to which the assay regions A1 and A2 are applied cannot be actually visually observed, the cutting position may deviate from the boundary between the first assay region A1 and the second assay region A2. As shown in Fig. 9, in a case where the cutting position deviates from the boundary between the first assay region A1 and the second assay region A2, for example, to the first assay region A1 side, the cutting is performed in the middle of the first assay region A1. In this way, in a case where the cutting is performed at a position deviated to the first assay region A1 side, as shown in the enlarged view of Fig. 9, the first assay region A1 is provided on both sides of the second assay region A2 in the width direction of one assay strip 1. In this way, in a case where the first assay regions A1 are provided on both sides of the second assay region A2, one half side in the width direction is originally the second assay region A2, and thus there is a concern that erroneous determination such as determination that the second assay region A2 is also positive even in a case where only the first assay region A1 is positive, may occur.

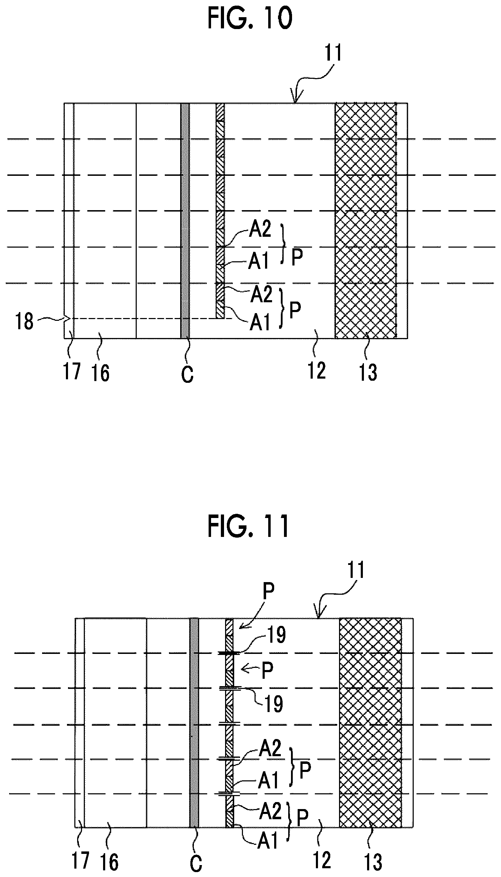

Therefore, it is preferable that the manufacturing method of an assay strip 1 includes a marking step of providing, on the sheet-shaped carrier 12, a mark 18 indicating a position to be cut in the cutting step, and the sheet-shaped carrier 12 is cut based on the mark 18. Examples of the mark 18 indicating a position to be cut in the cutting step include a notch as shown in Fig. 10. The mark 18 is not limited to a notch and may be a symbol, such as an arrow or a cross mark. Here, "providing the marks 18 on the sheet-shaped carrier 12" includes not only a case where the marks 18 are provided on the sheet-shaped carrier 12 itself, but also a case where the mark 18 is provided on a member other than the sheet-shaped carrier 12 of the sheet-shaped laminate 11, which indicates the cut portion of the sheet-shaped carrier 12, similarly to the case where the marks 18 are provided on the sheet-shaped carrier 12 itself. In the present example, the mark 18 is provided on the sheet-shaped substrate 17 to which the sheet-shaped carrier 12 is attached. The mark 18 is provided at a position related to the arrangement pattern P of the first assay region A1 and the second assay region A2, where one assay strip 1 is cut to include one arrangement pattern P.

Since the mark 18 indicating a position to be cut in the cutting step is provided on the sheet-shaped carrier 12 and the sheet-shaped carrier 12 is cut based on the mark 18, the cutting can be performed with high accuracy at the cutting position, which is located on the boundary between first assay region A1 and the second assay region A2, for cutting by one assay strip 1.

The mark 18 does not need to be provided on each of the plurality of cutting lines, and may be provided at one position indicating a cutting line to be cut first among the plurality of cutting lines. The mark 18 may be provided at a cutting start position of a cutting line to be cut first among the plurality of cutting lines, or may be provided at a portion to be a starting point indicating the cutting start position as shown in Fig. 10. In the case of Fig. 10, in a case where the width of the assay strip 1 is set as one period, the marks 18 are provided at positions deviated from the cutting start position by a half period.

It is preferable that the marking step is performed before the coating step. In this case, the mark 18 is used as the starting point of the cutting start position and can also be used as the starting point of the coating start position. As shown in Fig. 10, by determining a coating start position from the mark 18 as a starting point and determining a cutting start position from the mark 18 as a starting point, it is possible to accurately determine the cutting position.

In addition, as shown in Fig. 11, in the coating step, a gap region 19 where neither of the plurality of kinds of coating liquids may be applied is preferably provided between the arrangement patterns P adjacent to each other in the repetition direction of the arrangement pattern P of the first assay region A1 and the second assay region A2. That is, as shown in Fig. 11, it is preferable to provide the gap region 19 for each period of the arrangement pattern P of the first assay region A1 and the second assay region A2. Then, in the cutting step, the sheet-shaped laminate 11 including the sheet-shaped carrier 12 is preferably cut in the gap regions 19.

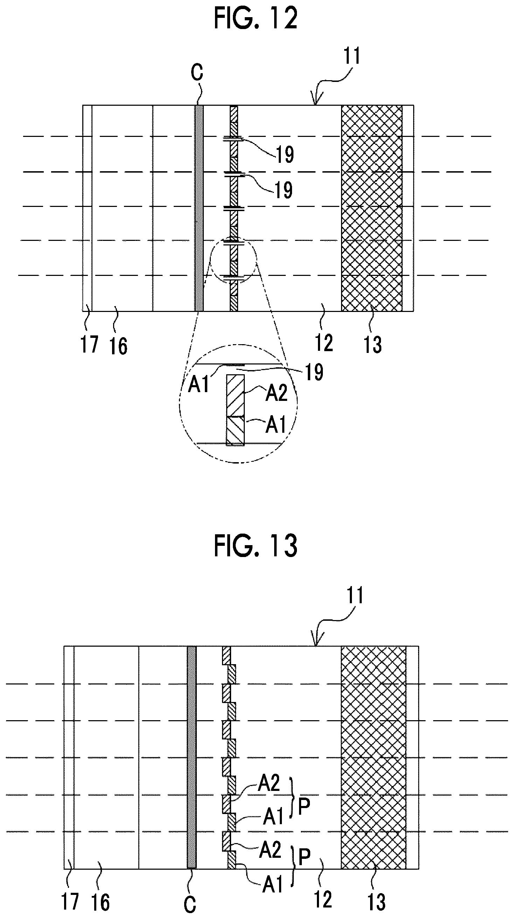

In this way, in a case where the gap regions 19 are provided for each period of the arrangement pattern P, it is possible to suppress the occurrence of an erroneous determination even in a case where a cutting position deviates. In the sheet-shaped carrier 12, one end part of the second assay region A2 is the gap region 19. Therefore, as shown in Fig. 12, even in a case where the cutting position deviates from the gap region 19 to the first assay region A1 side, in the width direction of one strip-shaped carrier 2, one end part of the second assay region A2 is adjacent to the gap region 19, and thus the first assay region A1 is not formed to be in contact with both sides of the second assay region A2. In addition, since one end part of the second assay region A2 is adjacent to the gap region 19, even in a case where the cutting position deviates within the first assay region A1, the end part of the assay strip 1 is slightly reached. Therefore, in a case where only the first assay region A1 is positive, the occurrence of erroneous determination such as determining that the second assay region A2 is positive can be suppressed.

In addition, in the coating step, a plurality of kinds of coating liquids may be applied in an aspect in which the positions of the adjacent assay regions between the arrangement patterns P adjacent to each other in a direction orthogonal to the repetition direction of the arrangement pattern P deviate from each other. For example, as shown in Fig. 13, the first assay region A1 and the second assay region A2 are arranged to be deviated in the longitudinal direction of the assay strip 1 cut out from the sheet-shaped laminate 11, whereby the positions of adjacent assay regions (here, the first assay region A1 and the second assay region A2) between the adjacent arrangement patterns P deviates. In the example shown in Fig. 13, the repetition direction of the arrangement pattern P is the width direction of the assay strip 1 cut out from the sheet-shaped laminate 11, and the direction orthogonal to the repetition direction is the longitudinal direction of the assay strip 1.

The repetition pattern of the arrangement pattern P shown in Fig. 13 is obtained by deviating, in the first coating step, the region to which the first coating liquid is applied and the region to which the second coating liquid is applied, in the longitudinal direction of the assay strip 1 cut out from the sheet-shaped laminate 11. More specifically, in a case proceeding to the second coating step (Step S14) after the first coating step (Step S13), the masking member 110 is deviated in the arrangement direction of the arrangement pattern, and is also deviated in the longitudinal direction of the assay strip 1. By applying the second coating liquid in this state, it is possible to apply a coating liquid containing the second binding substance 57 to a region deviated in the longitudinal direction of the assay strip 1 with respect to the region to which the coating liquid containing the first binding substance 56 is applied in the first coating step. Accordingly, as shown in Fig. 13, a repetition pattern can be obtained in which the arrangement pattern P in which the first assay region A1 and the second assay region A2 deviate from each other in the longitudinal direction of the strip-shaped carrier 2 is periodically repeated.

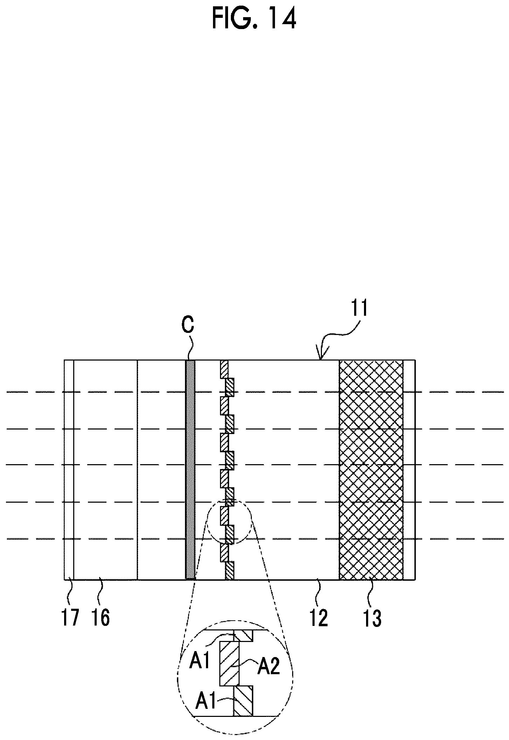

In the sheet-shaped carrier 12 shown in Fig. 13, the adjacent assay regions between the arrangement patterns P are arranged to be deviated from each other in a direction orthogonal to the repetition direction of the arrangement pattern P. As shown in Fig. 14 provided with such a repetition pattern of the assay regions, in a case where the cutting position deviates from the boundary between the arrangement patterns P to the first assay region A1 side of one arrangement pattern P, as shown in the enlarged view of Fig. 14, the first assay region A1 is provided at both end parts in the width direction on one strip-shaped carrier 2. As described with reference to Fig. 9, in a case where the first assay region A1 and the second assay region A2 coincide in the longitudinal direction of the assay strip 1 and are arranged linearly in parallel, there is a concern that the results of the assay region A1 and the second assay region A2 may be confused and an erroneous determination is made. However, as shown in the enlarged view of Fig. 14, even in a case where the first assay region A1 is provided at both end parts in the width direction of one strip-shaped carrier 2, the first assay region A1 and the second assay region A2 are arranged to be deviated from each other in a direction orthogonal to the repetition direction of the arrangement pattern P. Therefore, the identifiability of the first assay region A1 and the second assay region A2 is high and the occurrence of erroneous determination can be suppressed.

Here, although the case where the two assay regions A1 and A2 are provided in one arrangement pattern has been described, in a case where three or more assay regions are provided, regions formed at end part in the width direction of the assay strip 1 of the arrangement pattern may deviate from each other in the longitudinal direction. Accordingly, the adjacent assay regions of the adjacent arrangement patterns can be arranged to be deviated in the longitudinal direction. In a case where the adjacent assay regions of the adjacent arrangement patterns are arranged to be deviated in the longitudinal direction, since the boundary between the arrangement patterns is clear even in a case where the cutting positions have deviated from each other, erroneous determination can be suppressed.

In the above-described manufacturing method of the assay strip 1, although the first assay region A1 and the second assay region A2 are applied and formed by defining the coating region using the masking member 110, the forming method of the assay region is not limited to the coating method using the masking member 110. For example, application of a plurality of kinds of coating liquids may be performed by an inkjet method.

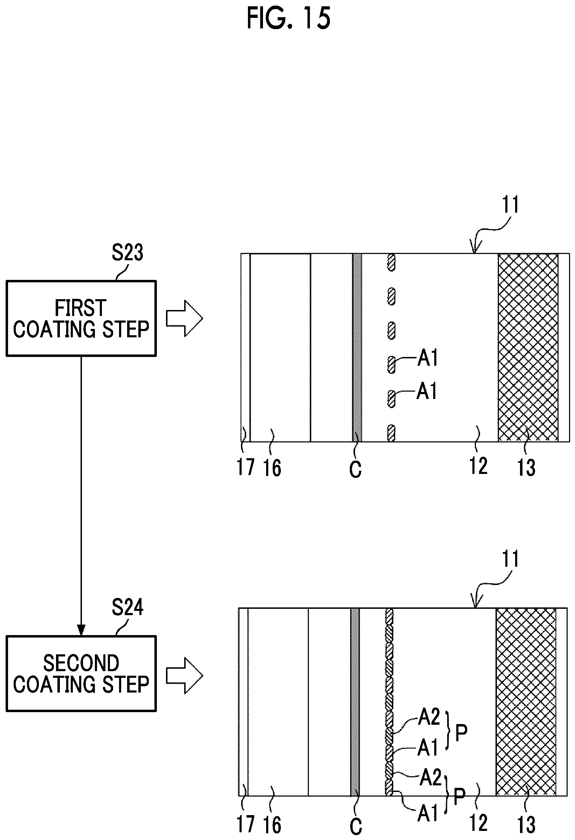

Fig. 15 is a diagram showing the first coating step and the second coating step in a case where the ink jet method is used. In the manufacturing method of an assay strip 1 described with reference to Fig. 6 to Fig. 8, the first coating step S13 and the second coating step S14 may be replaced with a first coating step S23 and a second coating step S24 using an inkjet method. The ink jet method is carried out using an ink jet coating device.

As shown in Fig. 15, in the first coating step S23, the first coating liquid is added dropwise onto the first assay region formation region to form the first assay region A1. The first assay region A1 is formed on a straight line parallel to the control region C with a period corresponding to one period of the arrangement pattern.
Then, in the second coating step S24, the second coating liquid is added dropwise onto the region between the first assay regions A1 to form the second assay region A2.

In this way, even in a case where the inkjet method is used, the assay strip 1 can be manufactured similarly to the case of using the masking member 110. According to the ink jet method, it is possible to form the assay region with high accuracy without using the masking member 110.

The disclosure of Japanese Patent Application No. 2021-120871 filed on July 21, 2021 is incorporated herein by reference in its entirety.

All literatures, patent applications, and technical standards described in the present specification are incorporated in the present specification by reference to the same extent as in a case where the individual literatures, patent applications, and technical standards are specifically and individually stated to be incorporated by reference.

## Claims

1. An assay cartridge that is used for immunochromatographic assay, the assay cartridge comprising:
an assay strip that has, as an assay region for determining whether a sample is positive or negative, a plurality of kinds of assay regions each on which a plurality of kinds of binding substances that respectively specifically bind to a plurality of different kinds of test substances is immobilized, and that includes a strip-shaped carrier capable of performing an assay of a plurality of kinds of assay items depending on the number of different kinds of the assay regions,
wherein in a case where a direction orthogonal to a longitudinal direction of the strip-shaped carrier is defined as a width direction,
the plurality of kinds of the assay regions has a length in the width direction of the strip-shaped carrier shorter than an entire width of the strip-shaped carrier, and
the plurality of kinds of the assay regions are arranged to be positionally deviated in the width direction.

2. The assay cartridge according to claim 1,
wherein the plurality of kinds of the assay regions are arranged side by side in the width direction of the strip-shaped carrier.

3. The assay cartridge according to claim 2,
wherein in the plurality of kinds of the assay regions, center positions in the longitudinal direction of the strip-shaped carrier coincide with each other, and widths are the same.

4. The assay cartridge according to claim 2,
wherein different kinds of the assay regions adjacent to each other in the width direction of the strip-shaped carrier are arranged to be partially deviated in the longitudinal direction of the strip-shaped carrier.

5. The assay cartridge according to claim 1,
wherein the plurality of kinds of the assay regions are arranged in a mode without overlapping each other in the longitudinal direction of the strip-shaped carrier.

6. The assay cartridge according to claim 5,
wherein in the longitudinal direction of the strip-shaped carrier, an interval between end parts of the plurality of kinds of assay regions is less than 2 mm.

7. A manufacturing method of the assay strip included in the assay cartridge according to any one of claims 1 to 6, the manufacturing method comprising:
a coating step of applying a plurality of kinds of coating liquids each containing the plurality of kinds of the binding substances to form the plurality of kinds of the assay regions on a sheet-shaped carrier having an area including a plurality of the strip-shaped carriers, in which in the sheet-shaped carrier, the plurality of kinds of the coating liquids are applied in a mode in which an arrangement pattern of the plurality of kinds of the assay regions in the one strip-shaped carrier is periodically repeated; and
a cutting step of cutting the sheet-shaped carrier along a direction orthogonal to a repetition direction of the arrangement pattern.

8. The manufacturing method of the assay strip according to claim 7,
wherein in the coating step, the plurality of kinds of the coating liquids are applied in a mode in which a plurality of the arrangement patterns is linearly arranged along the repetition direction.

9. The manufacturing method of the assay strip according to claim 8,
wherein in the coating step, a gap region to which none of the plurality of kinds of the coating liquids is applied is provided between adjacent arrangement patterns in the repetition direction, and
in the cutting step, the sheet-shaped carrier is cut in the gap region.

10. The manufacturing method of the assay strip according to claim 7,
wherein in the coating step, the plurality of kinds of the coating liquids are applied in a mode in which adjacent assay regions between adjacent arrangement patterns in the repetition direction are positionally shifted in the direction orthogonal to the repetition direction.

11. The manufacturing method of the assay strip according to any one of claims 7 to 10,
wherein a masking member including a plurality of opening portions linearly arranged along the repetition direction in a period in which a width of one strip-shaped carrier is defined as one period, in which a length of each of the opening portions in the repetition direction is the length of the assay region, is used, and
the coating step includes a first coating step of placing the masking member at a first position on the sheet-shaped carrier and applying a first coating liquid that is one of the plurality of kinds of coating liquids on a first part that is exposed from the opening portion, and includes a second coating step of moving the masking member from the first position to a second position by shifting in the repetition direction within the one period and applying a second coating liquid that is another one of the plurality of kinds of coating liquids on a second part that is exposed from the opening portion at the second position and to which the first coating liquid is not applied.

12. The manufacturing method of the assay strip according to any one of claims 7 to 10, wherein the plurality of kinds of the coating liquids are applied by an ink jet method.

13. The manufacturing method of the assay strip according to any one of claims 7 to 12, the manufacturing method further comprising:
a marking step of providing, on the sheet-shaped carrier, a mark indicating a position to be cut in the cutting step,
wherein in the cutting step, the sheet-shaped carrier is cut based on the mark.
